# EUROPÄISCHE PATENTSCHRIFT

(11) **EP 0 696 642 B1**
(45) Veröffentlichungstag und Bekanntmachung des Hinweises auf die Patenterteilung: **03.07.2002**
(21) Anmeldenummer: 95111554.2
(22) Anmeldetag: 22.07.1995
(51) Int. Cl.: C12Q 1/56

(54) **Verfahren zum Nachweis von Störungen des Protein C/Protein S-Systems**
Process for the detection of disorders of the protein C/protein S system
Procédé de détection de dérangements du système protéine C/protéine S

(30) Priorität: 08.08.1994 DE 4427785
(43) Veröffentlichungstag der Anmeldung: 14.02.1996
(73) Patentinhaber: Dade Behring Marburg GmbH, 35001 Marburg (DE)
(72) Erfinder: Kraus, Michael, Dr., D-35041 Marburg (DE)

(56) Entgegenhaltungen:
- EP-A- 0 236 985
- EP-A- 0 406 971
- WO-A-91/01382
- WO-A-93/10262

## Beschreibung

Die vorliegende Erfindung betrifft ein Verfahren zum Nachweis von Störungen des Protein C/Protein S-Systems.

Das Protein C/Protein S-System stellt einen wichtigen antikoagulatorischen Mechanismus dar. Im Normalfall stehen die koagulatorischen und antikoagulatorischen Mechanismen der Gerinnung in einem ausgewogenen Verhältnis zu einander.

Die Aktivierung der Gerinnung mündet in der Umsetzung des Proenzyms Prothrombin zur aktiven Protease Thrombin. Thrombin beschleunigt seine Entstehung selbst, in dem es die Kofaktoren Faktor V und Faktor VIII durch proteolytische Spaltung aktiviert. Diese aktivierten Kofaktoren bilden mit den Proteasen Faktor Xa bzw. IXa aktive Enzym/Kofaktor-Komplexe auf Phospholipidoberflächen, deren Aktivität um den Faktor ca. 1000 höher ist als die der singulären Proteasen. Durch diese positive Rückkopp-lung kommt es quasi explosionsartig zur Entstehung großer Mengen an Thrombin. Thrombin setzt Fibrinogen zu Fibrin um, das im Normalfall zum Wundverschluß und zur Wundheilung führt. Um eine lebensbedrohende Ausweitung der Gerinnung zu verhindern, die zu einem Verschluß des Gefäßsystems im Körper, also zu Thrombosen führen würde, müssen sowohl die aktive Protease als auch die Nachlieferung der Protease unterbunden werden. Aktive Proteasen werden im Körper durch Protease-Inhibitoren durch die Ausbildung kovalenter Komplexe neutralisiert. Die Unterbrechung des Nachschubes wird durch Thrombin selbst initiiert. Thrombin bindet hierzu an das Membranprotein Thrombomodulin und setzt das Proenzym Protein C (PC) zur aktiven Protease Protein Ca (APC) um. APC seinerseits bildet mit dem Kofaktor Protein S (PS) einen Komplex, der die aktiven Kofaktoren Faktor VIIIa und Faktor Va proteolytisch spaltet und dadurch inaktiviert. APC unterbricht somit die starke Stimulierung durch diese Kofaktoren.

Die Bedeutung des Protein C/Protein S-Systems wird dadurch bestätigt, daß Personen mit erblichen oder erworbenen Mängeln oder Defekten an Protein C und/oder Protein S mit hoher Wahrscheinlichkeit Thrombosen, insbesondere rezidivierende venöse Thrombosen, erleiden (Esmon, C.T., TCM 2: 214-219, 1992). Neben Protein C und Protein S können weitere Faktoren die Aktivität des Systems beeinflussen, so von Willebrand Faktor und Faktor IXa (Rick, M.E. et al., J. Lab. Clin. Med. 115: 415-421, 1990), die Faktor VIIIa vor proteolytischem Abbau schützen können. Erworbene Störungen können auch auf die Entstehung von Lupus anticoagulants zurückgehen. Dies sind gegen Phospholipide gerichtete Antikörper, die die zur Funktion notwendige Anbindung der Protease/Kofaktor Komplexe an Phospholipid-Oberflächen stören (Amer, L. et al., Thromb. Res. 57: 247-258, 1990). Schließlich wurde in jüngster Zeit eine Mutation des Faktors V beschrieben, der nicht mehr oder zumindest nur sehr schlecht durch APC inaktiviert werden kann (Bertina, R.M. et al., Nature 369: 64-67, 1994).

Aufgrund der vielen möglichen Störungen des für seine antithrombotische Wirkung wichtigen Protein C/Protein S Systems, ist es in der klinischen Diagnostik sinnvoll, einen Screening Test anzuwenden, der eine Störung in diesem System generell anzeigt. Dies gilt insbesondere dann, wenn bestimmte Störungen, wie in diesem Fall durch von Willebrand-Faktor, Faktor IXa, Lupus anticoagulant oder die Mutation des Faktors V, nur unter großem Aufwand in speziell darin erfahrenen Labors analysiert werden können. Darüber hinaus kann ein Screening Test, der das Prinzip der Wirkung des Protein C/Protein S Systems nutzt, auch Störungen anzeigen, deren Ursachen derzeit noch nicht bekannt sind.

Bisher wurden Protein C oder Protein S als Einzelfaktoren auf ihre Funktionalität untersucht. Hierzu wird die Probe oder aus der Probe isoliertes Protein C zunächst im Unterschuß zu einem Protein C-Mangelplasma zugesetzt. Die Aktivierung des Protein C erfolgt anschließend entweder durch Zusatz von Thrombin oder Thrombin und Thrombomodulin oder durch Zugabe eines Schlangengifts von *Agkistrodon contortrix,* das unter dem Handelsnamen Protac® (Fa. Pentapharm, Basel, Schweiz) bekannt ist. Die Detektion des in der Probe vorhanden Protein C erfolgt entweder anhand der Verlängerung der Gerinnungszeit durch den antikoagulatorischen Effekt des in der Probe vorhandenen Protein C oder durch Umsetzung eines für Thrombin spezifischen Substrats. Alternativ kann durch die Verwendung eines spezifischen Substrats für APC die Protein C-Aktivität nach Aktivierung mit Thrombin oder Protac® auch direkt chromogen bestimmt werden.

Die Protein S Bestimmungen erfolgen durch Mischung der Probe mit PS-Mangelplasma. Die stimulierende Wirkung des Protein S auf die antikoagulatorische Aktivität von APC wird durch die Bestimmung der Verlängerung derGerinnungszeit erfaßt. Das hierzu benötigte APC wird entweder zugegeben oder es wird mittels Protac® das Protein C im PS-Mangelplasma aktiviert. (eine Übersicht wird gegeben in Bertina, R. M., Res. Clin. Lab. 20: 127-138, 1990).

Die bisher beschriebenen Methoden sind nur dazu geeignet, Störungen des Protein C oder Protein S durch den jeweils singulär untersuchten Faktor zu detektieren. Sie eignen sich daher nicht als Screening Tests.

In einem weiteren Verfahren (Amer, L. et al., Thromb. Res. 57: 247-258, 1990) wird die aktivierte, partielle Thromboplastinzeit (APTT) modifiziert. Die APTT ist ein Standardverfahren zur Detektion von Gerinnungsstörungen, d.h. sie dient zur Erkennung von Blutungsneigungen. Nach Aktivierung des Probenplasmas mittels einer aktivierenden Oberfläche wird die Gerinnung bei Amer et al. durch gleichzeitige Zugabe von Calciumionen und APC gestartet. Die Gerinnungszeiten werden durch die antikoagulatorische Wirkung des exogen zugegebenen APC verlängert. Dieser Test erkennt somit bereits gewisse Störungen des Protein C/Protein S-Systems. Da APC exogen zugegeben wird, können allerdings Defekte oder Mängel des Protein C in der Probe nicht erkannt werden.

Basierend auf der Thromboplastinzeit, einem weiteren Standardverfahren in der Gerinnungsdiagnostik, aktivieren Duchemin, J. et al. (Thromb. Haemost. 71, 331-338, 1994) in einer Probe durch Zugabe von Thromboplastin und Calcium die Gerinnung. Das entstehende Thrombin aktiviert bei gleichzeitiger Zugabe von Thrombomodulin das Protein C in der Probe (endogen). In Abhängigkeit von der Funktionstüchtigkeit des Protein C/Protein S Systems wirkt APC der Entstehung von Thrombin entgegen. Nach 15 Minuten wird durch Komplexierung der Calciumionen die weitere Gerinnungs-aktivität unterbrochen und das entstandene Thrombin durch Umsatz eines spezifischen chromogenen Substrats bestimmt. Die entstandene Menge an Thrombin ist indirekt abhängig von der Funktionsfähigkeit des Protein C/Protein S-Systems.

Alle Störungen des Protein C/Protein S-Systems der Probe lassen sich erkennen, da das endogene Protein C aktiviert wird. Nachteilig ist jedoch insbesondere die lange Gesamtmeßzeit von 16 Minuten. Für einen routinemäßigen Einsatz als Screening Test ist eine solch zeitaufwendiger Test unvorteilhaft. Weiterhin werden mit Thromboplastin und Thrombomodulin zwei Membranproteine benötigt, deren Herstellung aufwendig und deren Stabilität, insbesondere bei Thrombomodulin, begrenzt ist. Schließlich wird in der Probe im ersten Schritt bereits ein Gerinnsel erzeugt, so daß dieses Verfahren nur in Kombination mit chromogenen Meßmethoden möglich ist, die den Umsatz des erzeugten Thrombins trotz der Anwesenweit des Gerinnels erfassen. Die traditionelle Meßmethodik, die die Entstehung des Fibringerinnsels erfaßt, ist damit nicht möglich.

Der vorliegenden Erfindung lag somit das technische Problem zugrunde, ein Verfahren bereit zu stellen, das geeignet ist, vollständig die Funktionalität des Protein C/Protein S-Systems zu erfassen und sowohl mit der traditionellen Meßtechnik - Erfassung der Entstehung eines Fibringerinnels - als auch mit Hilfe von chromogenen Substraten, ausgewertet werden kann.

Die Lösung dieses technischen Problems erfolgt durch die Bereitstellung der in den Patentansprüchen gekennzeichneten Ausführungsformen.

Es wurde überraschenderweise festgestellt, daß Störungen des Protein C/Protein S-Systems in einem funktionellen Gerinnungstest nachgewiesen werden können, wenn durch Zusatz eines Protein C Aktivators zur Probe endogenes Protein C der Probe aktiviert wird, wodurch die Gerinnungszeit, vermutlich aufgrund des Abbaus der aktivierten Kofaktoren Faktor Va und Faktor VIIIa, im Normalfall verlängert wird. Eine geringer ausgeprägte Verlängerung der Gerinnungszeit deutet auf Störungen in diesem natürlichen anti-koagulatorischen System hin, weshalb dieser Test insbesondere auch als Screening Test geeignet ist.

Im erfindungsgemäßen Verfahren wird das endogene Protein C der Probe genutzt, um die Funktionalität des Protein C/Protein S Systems zu überprüfen. Der Test basiert auf einer Modifikation der APTT. Zunächst wird ein Protein C-Aktivator, Kontaktphasen-aktivator, Phospholipide sowie die Probe in einem Testgefäß zusammengebracht und anschließend inkubiert. In dieser Phase werden, wie in der APTT üblich, die Proteasen Faktor XII, Präkallikrein und Faktor Xl aktiviert. Zusätzlich wird durch den Protein C-Aktivator das endogene Protein C in der Probe aktiviert, das mit dem Protein S der Probe auf den Phospholipidoberflächen aktive APC/Protein S Komplexe ausbildet. Nach der Inkubation wird die Gerinnung durch Zugabe von Calciumionen ausgelöst und die entstandenen APC/Protein S-Komplexe verzögern die Gerinnselbildung wie oben bereits beschrieben.

Im Unterschied zu Duchemin et al. wird im erfindungsgemäßen Verfahren Protein C nicht durch das Thrombin der Probe aktiviert, sondern durch Zugabe eines Protein C Aktivators. Dies erlaubt, die Meßzeit wesentlich zu reduzieren. So genügen, wie im Beispiel 1 aufgeführt, bereits kurze Inkubationszeiten von 2 Minuten vollkommen, um eine Aussage über die Funktionsfähigkeit des Protein C/Protein S-Systems treffen zu können. Schließlich erfolgt die Detektion über die Bestimmung der Gerinnungszeit, so daß sowohl traditionell (Bestimmung des Einsetzens der Gerinnselbildung), als auch chromogen (Umsetzung eines chromogenen Substrats) messende Geräte verwendet werden können.

Im Gegensatz zu den früheren Verfahren zur Protein C oder Protein S Bestimmung wird die Probe nicht mit einem entsprechenden Mangelplasma gemischt, so daß nur die Faktoren der Probe selbst in die Bestimmung mit eingehen. Schließlich zeigt das Beispiel 2, daß bei Zugabe von exogenem APC mit dem Verfahren nach Amer et al. (1990) ein Protein C Mangel nicht erkannt wird, während dies in Beispiel 1 mit dem erfindungsgemäßen Verfahren der Fall ist.

Aus der Sicht der Hämostaseologie drohen dem Organismus zwei Gefahren, durch die das Blut seine Funktion als Organ verlieren kann: zum einen der Blutverlust, zum anderen die intravasale Gerinnung. Dementsprechend stellt die Diagnostik Verfahren zur Detektion von Blutungsneigungen und Verfahren zur Detektion von Gerinnungsnei-gungen zur Verfügung. Das erfindungsgemäße Verfahren - zum Nachweis von Störungen des Protein C/Protein S-Systems - gehört zu der Klasse der Nachweis-verfahren zur Detektion einer Gerinnungsneigung. Überraschenderweise basiert das erfindungsgemäße Verfahren jedoch auf einer Modifikation eines Standardverfahrens zur Detektion von Blutungsneigungen, der aktivierten partiellen Thromboplastinzeit (APTT). Als Oberflächenaktivatoren der Kontaktphase des Gerinnungssystems können alle dem Stand der Technik entsprechenden Materialien, wie beispielsweise Kaolin, Silica, Glas oder Ellagsäure verwendet werden.

Die Aktivierung des Protein C der Probe erfolgt proteolytisch mit einem geeigneten Enzym. Bevorzugt sind solche Enzyme, die keine anderen Faktoren des Gerinnungs-systems - außer Protein C- aktivieren oder anderweitig beeinflussen. Besonders bevorzugt sind daher Protein C-Aktivatoren aus dem Gift von Schlangen, wie beispielsweise *Agkistrodon contortrix contortrix*, *Agkistrodon bilineatus* oder *Agkistrodon halys halys*.

Die Konzentration des Protein C-Aktivators wird dabei so gewählt, daß in Verbindung mit der Einwirkdauer auf die Protein C Aktivierung (Inkubationszeit) im Test eine geeignete Verlängerung der Gerinnungszeit im Plasma erzeugt wird. Geeignet ist eine Verlängerung der Gerinnungszeit gegenüber der Gerinnungszeit ohne Anwesenheit eines Protein C-Aktivators, die aufgrund des verwendeten Gerätetypus signifikante Abweichungen von normalen Plasmen erkennen läßt. Bevorzugterweise beträgt die Verlängerung mindestens 30 %, besonders bevorzugterweise mindestens 100 %, ganz besonders bevorzugterweise mindestens 200 %.

Es ist auch denkbar, daß das Protein C in der Probe vollständig durch vorhergehende Inkubation mit einem Protein C-Aktivator aktiviert wird. Dann muß allerdings die Probe entsprechend verdünnt werden oder es dürfen nur geringe Probenmengen verwendet werden, damit die Probe noch gerinnbar bleibt.

Benötigt der verwendete Protein C-Aktivator keine Phospholipide zur Aktivierung von Protein C, kann die Inkubation von Oberflächenaktivator, Probe und Protein C-Aktivator auch in Abwesenheit von Phospholipiden erfolgen und diese erst später zugesetzt werden. Die Reihenfolge der Zugabe von Oberflächenaktivator, Probe, Protein C-Aktivator und gegebenenfalls Phospholipiden kann variiert werden. Bevorzugt ist daher ein Reagenz, das eine aktivierende Oberfläche, Phospholipide und einen Protein C Aktivator enthält (siehe Beispiel 3). Besonders bevorzugt ist als weiterer Zusatz in einem Reagenz ein chromogenes Substrat für Thrombin, so daß die Bestimmung der Gerinnungszeit auch chromogen erfolgen kann (siehe Beispiel 4).

Weiterhin ist eine Kombination mit zusätzlichen Reagenzien möglich, die eine genauere Spezifikation des gefundenen Defekts erlauben. So kann im Fall eines Verdachts auf Protein C Mangel oder Defekt die Probe mit einer Protein C-haltigen Lösung gemischt werden. War ein Protein C Mangel oder Defekt tatsächlich die Ursache für eine abnorm verkürzte Gerinnungszeit im erfindungsgemäßen Verfahren, so wird sie durch den Zusatz neutralisiert (siehe Beispiel 5). Entsprechend kann Protein S bei Verdacht auf Protein S Mangel oder Defekt, Faktor V (F.V) oder eine Faktor V angereicherte Lösung bei Verdacht auf abnormen Faktor V bzw. Phospholipide zur Neutralisierung inhibitorischer Antikörper ("Lupus anticoagulant") zugesetzt werden. Bevorzugterweise werden diese Zusätze in einer solchen Menge zugegeben, daß in dem Testansatz jeweils die funktionelle Menge der jeweiligen Zusatzkomponente vorhanden ist, die sich auch mit einem Normalplasma ergeben würde. Diese Zusätze werden sinnvollerweise vor Zugabe des Protein C-Aktivators gegeben. Bevorzugt ist eine vorherige Inkubation der Zusätze mit der Probe. Besonders bevorzugt ist eine solche Vorinkubation bei Zugabe von Phospholipiden zur Neutralisierung von inhibitorischen Antikörpern.

Da die Methode auf einer Modifikation einer Standardmethode zur Erkennung einer Blutungsgefahr, beruht, können neben der APTT auch andere gängige Gerinnungsmethoden verwendet werden, wie die Thromboplastin-Zeit (PT; siehe Beispiel 6) oder die 'Russell's Viper Venom Time' (RVVT; siehe Beispiel 7). Die Thromboplastin Zeit beruht auf der Verwendung eines Reagenzes, das neben Phospholipiden und Calciumchlorid Gewebefaktor (Thromboplastin; 'tissue factor') enthält, der den sogenannten extrinsischen Weg der Gerinnung aktiviert. Bei der RVVT wird Schlangengift, bevorzugt der Spezies *Vipera russellii*, verwendet um die Gerinnungsfaktoren Faktor X und V zu aktivieren, die dann direkt ohne weitere Zwischenschritte Thrombin aus Prothrombin erzeugen. Bei beiden Methoden wird die Faktor VIII-abhängige Gerinnungskaskade umgangen. Schwankungen der Konzentration an Faktor VIII haben in der APTT den stärksten Einfluß.auf das Ergebnis, da zum einen der Faktor VIII als Cofaktor die Gerinnungsvorgänge um ca. den Faktor 1000 beschleunigt und zum anderen die Faktor VIII-Konzentrationen in Patienten gemeinhin stark schwanken können. Die Anwendung einer PT oder RVVT gegenüber der APTT resultiert somit in einer spezifischere Methode, die weniger von den prokoagulatorischen Faktoren, als vielmehr nur noch von den Faktoren des Protein C/Protein S-Systems abhängig ist (Beispiel 8).

Die folgenden Beispiele sollen lediglich die Erfindung erläutern, jedoch die Ansprüche in keiner Weise einschränken.

### Beispiel 1

### Bestimmung der Gerinnungszeit unter Aktivierung des endogenen Protein C.

Die Bestimmung der Gerinnungszeit erfolgte an einem mechanischen Koagulometer nach Schnitger & Gross (Fa. Amelung). Alle Reagenzien waren Handelsprodukte der Fa. Behringwerke AG oder übliche Laborchemikalien. Folgende Proben wurden untersucht: ein Pool aus normalen Blutspendern (Standard-Human-Plasma), Protein C-Mangelplasma, Protein S-Mangelplasma und ein Plasma mit einem genetischen Defekt im Faktor V, so daß entstehender Faktor Va durch APC nur schlecht inaktiviert wird.

Eine Abfüllung Protein C-Aktivator für Berichrom® Protein C (enthält Protein C-Aktivator aus dem Gift von *Agkistrodon contortrix contortrix*) wurde in 10 ml physiologischer Kochsalzlösung gelöst. Pathromtin®, ein Phospholipidgemisch aus humaner Plazenta, wurde in 5 ml Kaolinsuspension als Oberflächenaktivator gelöst. Die Calciumchloridlösung (25 mM) und die Protein C-Aktivatorlösung wurden vor Gebrauch auf +37°C erwärmt.

In ein Meßröhrchen wurden nacheinander pipettiert
100 µl Protein C-Aktivator-Lösung
100 µl Pathromtin®
100 µl Plasmaprobe.

Anschließend wurde bei +37°C für 2 Minuten inkubiert und durch Zugabe von 100 µl Calciumchlorid-Lösung die Gerinnungszeit gestartet. Gleichzeitig wurde eine eingebaute Stoppuhr eingeschalten und die Zeit erfaßt, bis ein Gerinnsel detektiert wurde.

Weiterhin wurde die Gerinnungszeit der Proben ohne Aktivierung von Protein C, d.h. bei Zugabe von 100 µl physiologischer Kochsalzlösung anstelle der Protein C-Aktivator Lösung bestimmt.

In Tabelle 1 sind die erhaltenen Gerinnungszeiten (Mittelwerte aus Doppelbestimmungen) zusammengefaßt. Mit einem Pool von Citrat-Plasmen normaler Blutspender (SHP) wurden Gerinnungszeiten von ca. 42 sec. ohne und von 145 sec. mit PC-Aktivator erhalten. Diese durch die Aktivität des Protein C/Protein S-System erzielte Verlängerung von ca. 103 sec., war in den Proben, die einen Protein C- (PC-MP) oder Protein S-Mangel (PS-MP) aufwiesen, deutlich geringer. Auch das Plasma mit einem mutierten Faktor V (F.V-D) zeigte eine deutlich geringere Verlängerung der Gerinnungszeit.

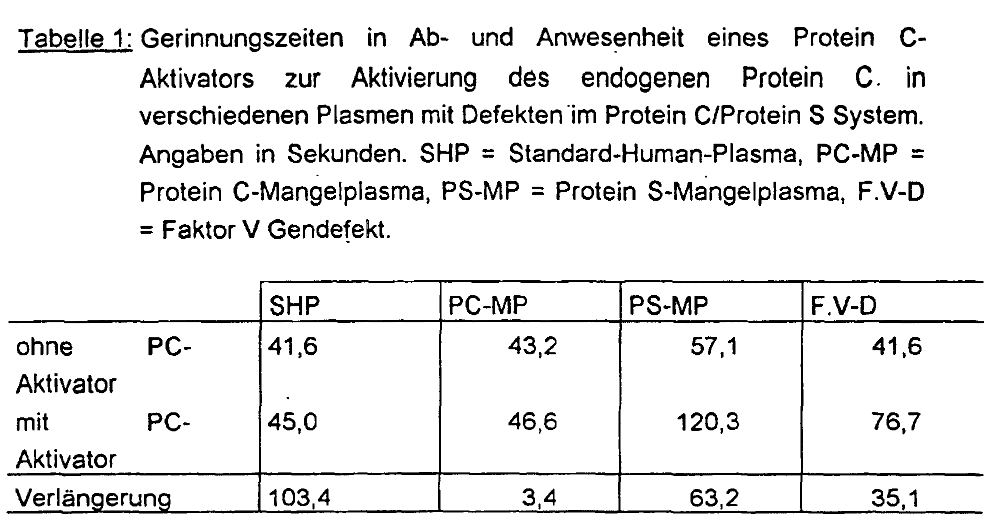

### Beispiel 2

### Bestimmung der Gerinnungszeit in Anwesenheit von exogenem Protein C.

Die Bestimmung der Gerinnungszeit erfolgte an einem mechanischen Koagulometer nach Schnitger & Gross (Fa. Amelung). Alle Reagenzien waren Handelsprodukte der Fa. Behringwerke AG oder übliche Laborchemikalien. Die Proben entsprechen denjenigen aus Beispiel 1.

Eine Abfüllung APC-Reagenz für APC-Sensitivitäts-Reagenzien (enthält humanes, aktiviertes Protein C und Calciumchlorid) wurde in 5 ml Aqua dest. gelöst. Pathromtin® diente wie unter Beispiel 1 als Oberflächenaktivator und Phospholipidgemisch. Das APC-Reagenz wurde vor Gebrauch auf +37°C erwärmt.

In ein Meßröhrchen wurden nacheinander pipettiert
100 µl Pathromtin®
100 µl Plasmaprobe.

Anschließend wurde bei +37°C für 2 Minuten inkubiert und durch Zugabe von 100 µl APC-Reagenz die Gerinnungszeit gestartet. Gleichzeitig wurde eine eingebaute Stoppuhr eingeschalten und die Zeit erfaßt, bis ein Gerinnsel detektiert wurde.

Weiterhin wurde die Gerinnungszeit der Proben ohne Aktivierung von Protein C, d.h. bei Zugabe von 100 µl Calciumchlorid-Lösung anstelle von APC-Reagenz bestimmt.

In Tabelle 2 sind die erhaltenen Gerinnungszeiten (Mittelwerte aus Doppelbestimmungen) zusammengefaßt. Mit einem Pool von Citrat-Plasmen normaler Blutspender (SHP) wurden Gerinnungszeiten von ca. 37 sec. ohne und von ca. 170 sec. in Anwesenheit von exogen zugeführten APC erhalten. Diese damit erzielte Verlängerung von ca. 133 sec. war in dem Plasma Protein S-Mangel (PS-MP), besonders aber in dem Plasma mit einem mutierten Faktor V (F.V-D) deutlich geringer. Hingegen wurde in diesem Verfahren in Gegensatz zum erfindungsgemäßen Verfahren ein Protein C Mangel nicht erkannt. Im Gegenteil, aufgrund des leichten Faktorenmangels, erkennbar an der etwas verlängerten APTT (= Gerinnungszeit ohne APC), wurde die Gerinnungszeit in Anwesenheit von APC noch deutlich über die des SHP hinaus verlängert. Dieses Verfahren ist im Gegensatz zum erfindungsgemäßen Verfahren nicht geeignet alle Defekte des Protein C/Protein S-Systems zu erkennen.

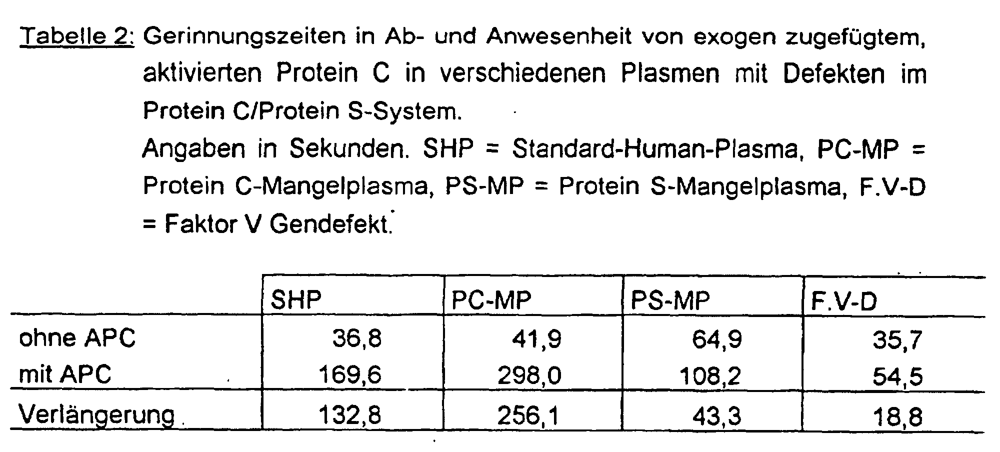

### Beispiel 3

### Bestimmung der Gerinnungszeit unter Aktivierung des endogenen Protein C bei Verwendung eines Monoreagenzes.

Die Bestimmung der Gerinnungszeit erfolgte an einem mechanischen Koagulometer nach Schnitger & Gross (Fa. Amelung). Alle Reagenzien waren von der Fa. Behringwerke AG. Erweiterend zu den Proben entsprechend aus Beispiel 1 wurden ein Faktor V-Mangelplasma (F.V-MP) und ein Faktor VIII-Mangelplasma (F.VIII-MP) mit untersucht.

Zwei Abfüllungen Protein C-Aktivator für Berichrom® Protein C wurden in je 2,5 ml Kaolinsuspension gelöst. Diese Lösungen (5 ml) wurden ihrerseits zum Lösen von 1 Abfüllung Pathromtin® verwendet. Dieses Monoreagenz enthält somit Phospholipide, Kaolin als Oberflächenaktivator und einen Aktivator für Protein C. Die Calciumchloridlösung (25 mM) und das Monoreagenz wurden vor Gebrauch auf +37°C erwärmt.

In ein Meßröhrchen wurden nacheinander pipettiert
100 µl Monoreagenz
100 µl Plasmaprobe

Anschließend wurde bei +37°C für 2 Minuten inkubiert und durch Zugabe von 100 µl Calciumchlorid-Lösung die Gerinnungszeit gestartet. Gleichzeitig wurde eine eingebaute Stoppuhr eingeschalten und die Zeit erfaßt bis ein Gerinnsel detektiert wurde.

Weiterhin wurde die Gerinnungszeit der Proben ohne Aktivierung von Protein C, d.h. bei Verwendung des kommerziellen Pathromtin® bestimmt.

In Tabelle 3 sind die Gerinnungszeiten (Mittelwerte aus Doppelbestimmungen) zusammengefaßt, die mit den verschiedenen Plasmen erhalten wurden. Mit einem Pool von Citrat-Plasmen normaler Blutspender (SHP) wurden Gerinnungszeiten von 37,6 sec. ohne und von 131,7 sec. mit PC-Aktivator im APTT-Reagenz erhalten. Analog zu den in Beispiel 1 aufgeführten Ergebnissen waren die Verlängerungen der Gerinnungszeit in einem Protein C- (PC-MP), einem Protein S-Mangel (PS-MP) oder in einem Plasma mit einem mutierten Faktor V (F.V-D) deutlich geringer. Dieses Beispiel zeigt auch, daß ein Mangel von Faktoren, der primär nicht mit dem Protein C/Protein S System, sondern mit der Thrombingenerierung zusammenhängt, etwa ein Faktor V- oder ein Faktor VIII-Mangel, in Anwesenheit eines Protein C Aktivators nicht zu einer Verkürzung, sondern im Gegenteil zu einer Verlängerung der Gerinnungszeit führt. Diese Plasmen sind dann nicht mehr gerinnbar, d.h. die Messung wurde nach 300 sec abgebrochen (">300" in Tabelle 3).

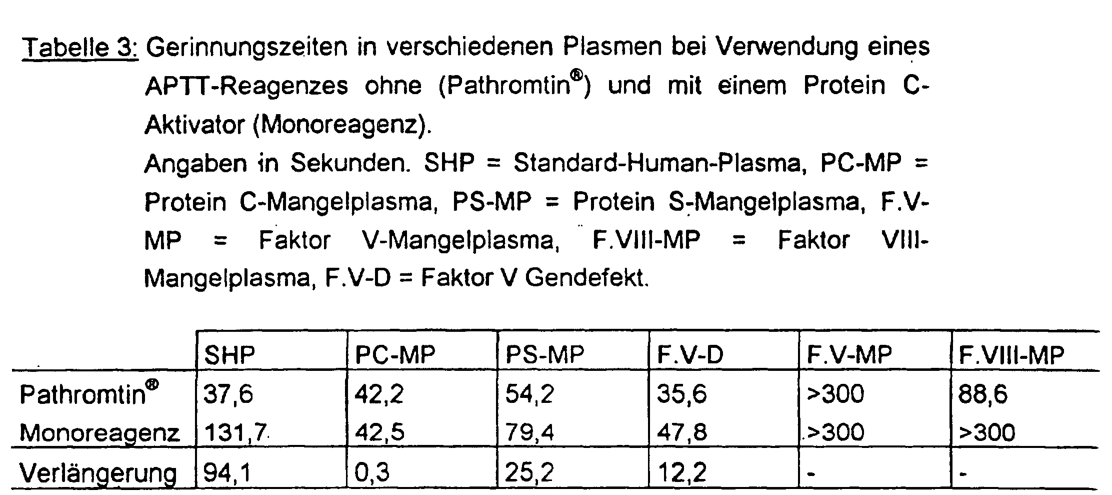

### Beispiel 4

### Bestimmung der Gerinnungszeit unter Aktivierung des endogenen Protein C bei Verwendung eines chromogenen Substrats für Thrombin.

Die Bestimmung der Gerinnungszeit erfolgte an einem Behring Coagulation Timer (BCT; Fa. Behringwerke), einem photometrischen Koagulometer. Alle Reagenzien waren von der Fa. Behringwerke AG. Die Proben entsprechen denjenigen aus Beispiel 1.

Eine Abfüllung Protein C-Aktivator für Berichrom® Protein C wurde in 10 ml physiologischer Kochsalzlösung gelöst. Als APTT-Aktivatorreagenz wurde Pathromtin® SL verwendet. Dies ist eine Suspension von Phospholipiden aus Sojabohnen mit Silica-Partikeln als Oberflächenaktivator. Als Startreagenz wurde ein Gemisch aus Calciumchloridlösung (25 mM) und 0,2 mM BCP-100, ein chromogenes Thrombin-Substrat, verwendet.

Die Pipettierung wurde vom Gerät selbständig durchgeführt. Das Startreagenz und die Protein C-Aktivatorlösung werden vom Gerät direkt vor Gebrauch auf +37°C erwärmt.

In eine Meßküvette wurden nacheinander pipettiert
70 µl Pathromtin® SL
10 µl Protein C-Aktivator bzw. nur physiologische Kochsalzlösung
70 µl Probe

Anschließend wurde bei +37°C für 2 Minuten inkubiert und durch Zugabe von 70 µl Startreagenz die Messung gestartet. Es wurde die Zeit registriert, zu der eine Zunahme der Extinktion von 0,3 bei 405 nm erreicht wurde.

In Tabelle 4 sind die erhaltenen Zeiten mit den verschiedenen Plasmen aufgeführt. Analog zu der klassischen Gerinnungsmethode (Erfassung der Bildung eines Fibringerinnsels) werden auch bei Verwendung eines chromogenen Substrats alle Plasmen mit einer Störung im Protein C/Protein S System gegenüber einem normalen Plasma Pool verkürzt gefunden.

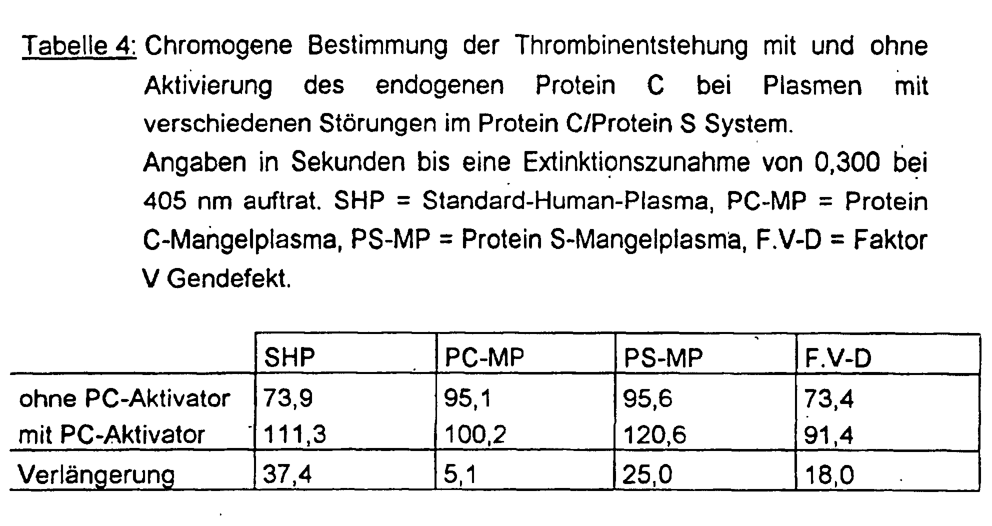

### Beispiel 5

### Identifizierung eines Defekts im Protein C/Protein S System durch Modifikation des Screening Verfahrens.

Wie unter Beispiel 3 und in Beispiel 4 beschrieben wurde die Gerinnungszeit einmal durch Erfassung der Gerinnselbildung mit einem Monoreagenz bzw. durch Erfassung der Thrombinbildung in einem getrennten Ansatz bestimmt. Dieser Screening-Test eignet sich auch zur Differentialdiagnostik. Dazu wurden die in Beispiel 3 und 4 aufgeführten Ansätze wie folgt modifiziert. Als erster Schritt wurden 5 µl einer Protein C-haltigen Lösung pipettiert. Die Konzentration wurde so gewählt, daß die resultierende Protein C-Konzentration bezogen auf das Probenvolumen 1 Einheit betrug. Danach erfolgt die normale Zugabe von Probe und Reagenzien.

Aus Tabelle 5 geht hervor, daß mit verschiedenen methodischen Varianten des Bestimmungsverfahrens ein Protein C-Mangel eindeutig etwa gegen einen Protein S-Mangel zu differenzieren ist. Während der Zusatz von Protein C zu einem Protein C-Mangelplasma die Meßdifferenz der Ansätze mit und ohne Zugabe eines Protein C-Aktivators kompensierte, war dies bei einem Protein S-Mangelplasma nicht der Fall. Somit ist dieses Verfahren durch leichte Modifikationen auch differentialdiagnostisch verwendbar.

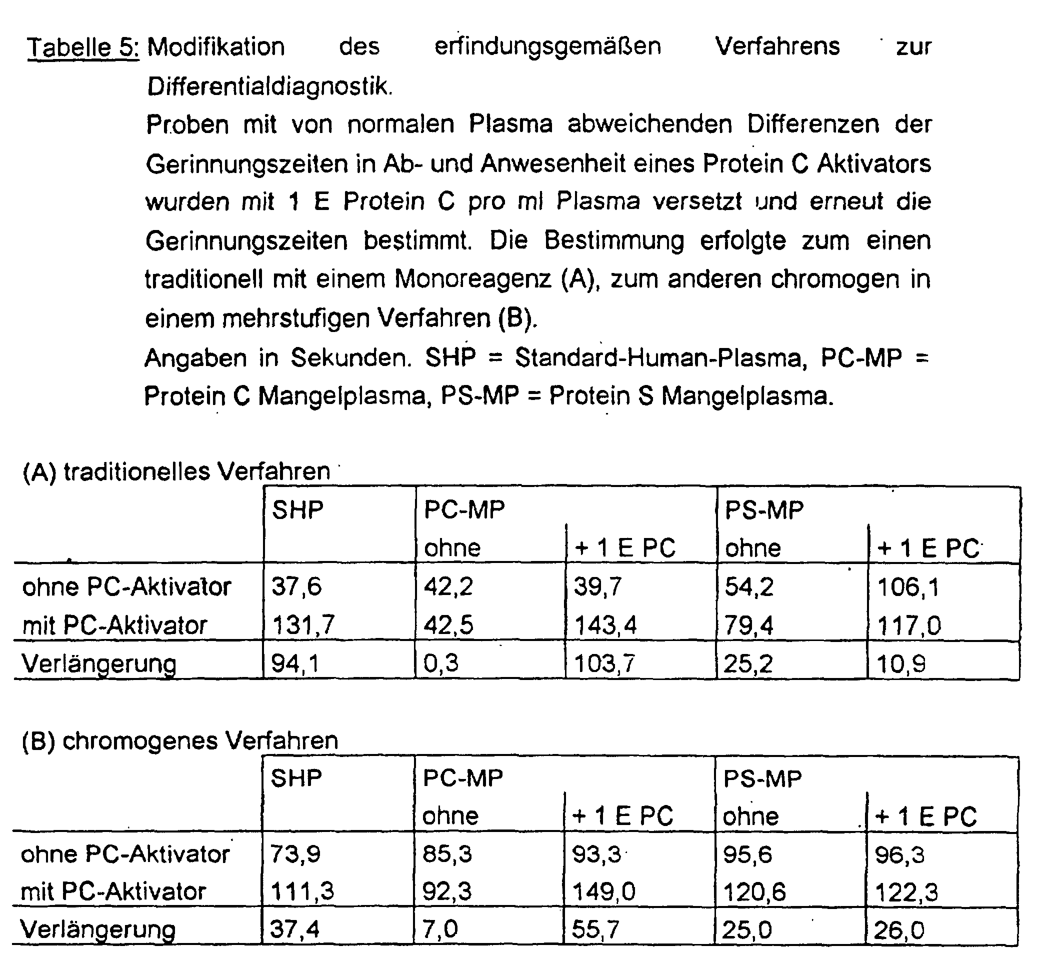

### Beispiel 6

### Bestimmung der Gerinnungszeit unter Aktivierung des endogenen Protein C bei Anwendung der Thromboplastinzeit.

Die Bestimmung der Gerinnungszeit erfolgte an einem mechanischen Koagulometer nach Schnitger & Gross (Fa. Amelung). Alle Reagenzien waren von der Fa. Behringwerke AG.

Thromborel S®, eine Gewebefaktor/Phospholipid-Präparation aus humaner Plazenta, wurde 1:1000 in 50 mM Tris-Puffer, pH 7,4, 0,01% Phospholipon-25, verdünnt. 5 ml dieser Lösung wurden verwendet um 1 Abfüllung Protein C-Aktivator für Berichrom Protein C, wie in Beispiel 3 beschrieben zu lösen.

Diese Lösung wurde vor Gebrauch auf +37°C erwärmt und anstelle des Monoreagenzes auf APTT-Basis, wie in Beispiel 3 beschrieben, verwendet.

Weiterhin wurde die Gerinnungszeit der Proben ohne Aktivierung von Protein C, d.h. bei Verwendung des kommerziellen Pathromtin® bestimmt.

In Tabelle 6 sind die Gerinnungszeiten (Mittelwerte aus Doppelbestimmungen) zusammengefaßt, die mit den verschiedenen Plasmen erhalten wurden. Analog zu den in Beispiel 1 aufgeführten Ergebnissen waren die Verlängerungen der Gerinnungszeit in einem Protein C- (PC-MP), einem Protein S-Mangel (PS-MP) oder in einem Plasma mit einem mutierten Faktor V (F.V-D) deutlich geringer.

**Tabelle 6:**

| | | | |
|---|---|---|---|
| Gerinnungszeiten in verschiedenen Plasmen bei Verwendung eines PT-Reagenzes (Thromborel S®) mit einem Protein C-Aktivator (Monoreagenz). Angaben in Sekunden. SHP = Standard-Human-Plasma, PC-MP = Protein C-Mangelplasma, PS-MP = Protein S-Mangelplasma,, F.V-D = Faktor V Gendefekt. | | | |

| SHP | PC-MP | PS-MP | F.V-D |
|---|---|---|---|
| 103,6 | 49,6 | 54,5 | 69,9 |

### Beispiel 7

### Bestimmung der Gerinnungszeit unter Aktivierung des endogenen Protein C bei Anwendung der RVVT.

Die Bestimmung der Gerinnungszeit an einem Behring Coagulation Timer (BCT; Fa. Behringwerke), einem photometrischen Koagulometer. Als RVVT-Reagenz wurde LA-Confirm, der Fa. Gradipore LTD (North Ryde, NSW, Australien) verwendet. Alle sonstigen Reagenzien waren von der Fa. Behringwerke AG. Die Proben entsprechen denjenigen aus Beispiel 1.

Eine Abfüllung Protein C-Aktivator für Berichrom® Protein C wurde in 10 ml physiologischer Kochsalzlösung gelöst. Das RVVT-Reagenz wurde nach Vorschrift gelöst. Alle Reagenzien wurden vor Gebrauch durch das Gerät auf +37°C gebracht.

In eine Meßküvette wurden nacheinander pipettiert
50 µl Probe
50 µl Protein C-Aktivator bzw. nur physiologische Kochsalzlösung

Anschließend wurde bei +37°C für 2 Minuten inkubiert und durch Zugabe von 100 µl RVVT-Reagenz die Messung gestartet. Es wurde die Zeit registriert, zu der eine Zunahme der Extinktion von 0,3 bei 405 nm erreicht wurde.

In Tabelle 7 sind die erhaltenen Zeiten mit den verschiedenen Plasmen aufgeführt. Analog zu den sonstigen klassischen Gerinnungsmethoden (APTT, PT) werden auch bei Verwendung eines RVVT-Reagenzes alle Plasmen mit einer Störung im Protein C/Protein S System gegenüber einem normalen Plasma Pool verkürzt gefunden.

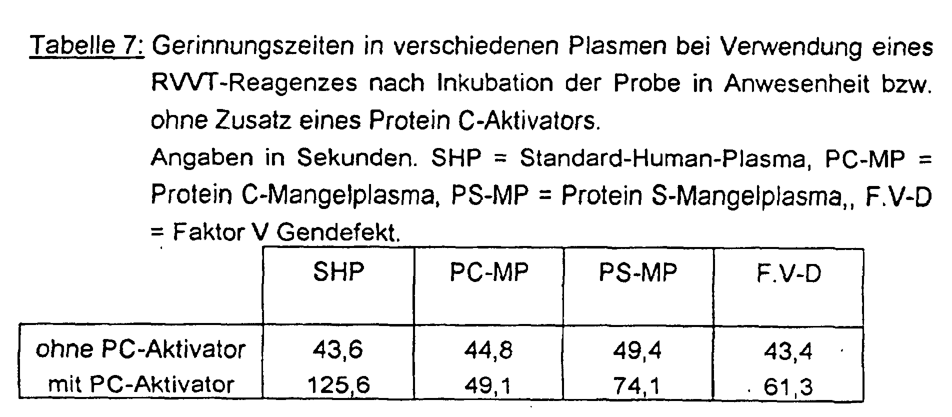

### Beispiel 8

### Abhängigkeit der Bestimmung von Faktor VIII bei Verwendung der APTT bzw. der PT unter Aktivierung des endogenen Protein C

Ein Standardhumanplasma wurde mit einem Faktor VIII-Mangelplasma gemischt bzw. Beriate®, ein Faktor VIII-Konzentrat (Behringwerke AG) zugegeben, um den Einfluß von Faktor VIII auf die Bestimmung der Gerinnungszeit nach Aktivierung des endogenen Protein C zu simulieren. Die Bestimmung unter Anwendung einer APTT erfolgte wie in Beispiel 1 beschrieben, die Bestimmung mittels einer PT erfolgte wie in Beispiel 6 beschrieben.

In Tabelle 8 sind die Gerinnungszeiten aufgeführt, die basierend auf einer APTT bzw. PT erhalten wurden. Es ist zu sehen, daß die unerwünschte Störung von Faktor VIII bei der Evaluierung der Funktionalität des Protein C-Systems bei der APTT deutlich ist, während sie in der PT-basierenden Methode praktisch nicht auftritt.

**Tabelle 8:**

| | | |
|---|---|---|
| Gerinnungszeiten in Abhängigkeit von der Faktor VIII-Konzentration bei Verwendung eines APTT bzw. eines PT-Reagenzes nach Inkubation der Probe mit einem Protein C-Aktivator. (Angaben in sec.) | | |

| Faktor VIII (E/ml) | APTT | PT |
|---|---|---|
| 0,25 | 206 | 114 |
| 0,5 | 147 | 111 |
| 1,0 | 96 | 104 |
| 1,5 | 79 | 102 |
| 2,0 | 65 | 109 |
| 3,0 | 60 | 107 |
| 4,0 | 54 | 107 |

## Patentansprüche

1. Screening Test zum Nachweis von Störungen des Protein C/Protein S-System der Gerinnung in einer Probe einer biologischen Flüssigkeit, der folgende Schritte einschließt:
a) Zugabe eines Aktivators des Protein C zu der verdünnten oder unverdünnten Probe; wobei die Probe nicht mit einem Protein C oder Protein S Mangelplasma gemischt wird;
b) optionale Zugabe eines Kontaktphaseaktivators;
c) Inkubation des Reaktionsansatzes;
d) Start des Gerinnungsablaufes durch Zugabe von Kalziumionen und/oder anderen Gerinnungs-auslösenden Agenzien und
e) Bestimmung der Gerinnungsaktivität.

2. Verfahren nach Anspruch 1, wobei der Protein C Aktivator aus der Gruppe der Protein C Aktivatoren ausgesucht wird, die überwiegend oder ausschließlich Protein C aktivieren, bevorzugterweise aus der Gruppe der Schlangengiftenzyme, besonders bevorzugterweise das Gift der Gattung *Agkistrodon*.

3. Verfahren nach mindestens einem der Ansprüche 1-2, wobei es sich bei dem Verfahren um eine Modifikation einer Standard methode zur Detektion von Blutungsneigungen handelt wie die Bestimmung der aktivierten, partiellen Thromboplastinzeit (APTT), der Thromboplastin-Zeit (PT) oder der Russell's Viper Venom Zeit (RVVT).

4. Verfahren nach mindestens einem der Ansprüche 1 - 3, wobei der Kontaktphaseaktivator ausgewählt wird aus der Gruppe die Kaolin, Silica, Glas und Ellagsäure umfaßt.

5. Verfahren nach mindestens einem der Ansprüche 1 - 4, wobei mindestens die Inkubation in Schritt c) bei kontrollierten Temperaturen abläuft, bevorzugterweise bei 36 - 38 °C.

6. Verfahren nach mindestens einem der Ansprüche 1 - 5, wobei die Konzentration des Protein C Aktivators so eingestellt wird, daß die Gerinnungszeit für ein Normalplasma um mindestens 30 %, bevorzugterweise mindestens 100 %, ganz bevorzugterweise mindestens 200 % verlängert wird.

7. Verfahren nach mindestens einem der Ansprüche 1 - 6, wobei die Inkubationsdauer in Schritt c) so eingestellt wird, daß die Gerinnungszeit für ein Normalplasma um mindestens 30 %, bevorzugterweise mindestens 100 %, ganz bevorzugterweise mindestens 200 % verlängert wird.

8. Verfahren nach mindestens einem der Ansprüche 1 - 7, wobei zwischen Schritt a) und b) eine weitere Inkubation erfolgt.

9. Verfahren nach mindestens einem der Ansprüche 1 - 8, wobei
i) die Zugabe eines Aktivators des Protein C,
ii) die Zugabe eines Kontaktphasenaktivators und
iii) der Start der Gerinnungsablaufs durch Zugabe von Calciumionen
gleichzeitig oder in kurzen Zeitabständen nacheinander erfolgt.

10. Verfahren nach mindestens einem der Ansprüche 1 - 9, wobei in Schritt e) ein chromogenes Substrat zur Bestimmung der Gerinnungsaktivität verwendet wird.

11. Verfahren nach mindestens einem der Ansprüche 1 - 10, wobei dem Ansatz vor Schritt c) Phospholipide hinzugefügt werden.

12. Verfahren nach mindestens einem der Ansprüche 1 - 10, wobei dem Ansatz nach Schritt c) Phospholipide hinzugefügt werden.

13. Verfahren nach mindestens einem der Ansprüche 11 und 12, wobei die Phospholipide aus der Gruppe der Phospholipide ausgewählt werden, die die Anlagerung von Enzym/Kofaktor Komplexen an die entstehenden Oberflächen bewirken.

14. Verfahren nach mindestens einem der Ansprüche 1 - 13, wobei in Schritt e) die Zeit bis zur Entstehung eines durch mechanische, mechano-optische oder turbidimetrische Meßmethoden detektierbares Gerinnsel zur Bestimmung der Gerinnungsaktivität verwendet wird.

15. Verfahren nach Anspruch 10, wobei in Schritt e) die Umsetzung eines chromogenen Substrats für Thrombin zur photometrischen Bestimmung der Gerinnungsaktivität verwendet wird.

16. Mittel zur Verwendung in einem Verfahren nach mindestens einem der Ansprüche 1 - 9, wobei das Reagenz den Protein C Aktivator, den Kontaktphaseaktivator und Phospholipide in einer in der in vitro Diagnostik üblichen Zubereitung enthält, **dadurch gekennzeichnet, dass** die Konzentration des Protein C Aktivators so gewählt wird, dass bei normalen Plasmen eine Verlängerung der Gerinnungszeit gegenüber der Gerinnungszeit ohne Anwesenheit eines Protein C Aktivators mindestens 30%, besonders bevorzugt mindestens 100%, ganz besonders bevorzugt mindestens 200%, beträgt.

17. Testkit zur Verwendung in einem Verfahren nach mindestens einem der Ansprüche 1 -15, das mindestens ein Mittel nach Anspruch 16 enthält.

18. Verfahren, **dadurch gekennzeichnet, dass** bei Proben, die in einem Verfahren gemäß der Ansprüche 1-3 von normalen Plasma abweichende Differenzen der Gerinnungszeiter aufweisen, die Gerinnungszeiten erneut in einem Verfahren gemäß der Ansprüche 1-3 bestimmt werden, wobei in einem der Schritte a) bis e) weitere Komponenten zugegeben werden, die geeignet sind, endogene Defizite im Protein C/ Protein S System auszugleichen.

19. Verfahren nach Anspruch 18, wobei die weiteren Komponenten ausgewählt sind aus der Gruppe: Protein C, Protein S, Faktor V oder Phospholipide.

20. Verfahren nach Anspruch 19, wobei die eingesetzten Proteine humanen Ursprungs sind.

21. Verfahren nach Anspruch 19, wobei die eingesetzten Proteine tierischen Ursprungs sind.

22. Verfahren nach mindestens einem der Ansprüche 18-21, wobei die Konzentration der weiteren Komponenten so gewählt ist, daß sie in geeigneter Mischung mit der Probe eine Störung des Protein C/Protein S-Systems aufgrund eines Mangels oder Defekts an diesen weiteren Komponenten ausgleichen.

23. Verfahren nach Anspruch 22, wobei die Konzentration der weiteren. Komponenten so gewählt ist, daß nach Mischung in der Probe diejenige funktionelle Stoffmenge vorhanden ist, die in einem normalen Plasma zu finden ist.

24. Verfahren nach Anspruch 22, wobei die weitere Komponente Faktor V ist und die Konzentration an Faktor V so gewählt ist, daß nach Mischung in der Probe ein mindestens zweifacher Überschuß an der Menge von funktionellem Faktor V vorhanden ist, die in einem normalen Plasma zu finden ist.

25. Verfahren nach mindestens einem der Ansprüche 18-24, wobei die Mischung der Probe mit der oder den weiteren Komponenten vor Zugabe des Protein C Aktivators erfolgt, bevorzugterweise wird die Probe mit der oder den Zusatzkomponenten vor Zugabe des Protein C Aktivators für eine Zeit von 1-10 min inkubiert.

26. Verfahren nach mindestens einem der Ansprüche 1-3, wobei auf einen Kontaktphase-Aktivator verzichtet wird und die Gerinnung auf Basis der Thromboplastin-Zeit mit Reagenzien ausgelöst werden kann, die Gewebefaktor und Phospholipide beinhalten

27. Verfahren nach Anspruch 26, wobei der Gewebefaktor natürlichen oder rekombinanten Ursprungs sein kann.

28. Verfahren nach mindestens einem der Ansprüche 26 und 27, wobei das Gewebefaktor-haltige Reagenz bereits einen Protein C-Aktivator enthält oder die Zugabe des Gewebefaktor getrennt von der Zugabe des Protein C-Aktivators erfolgt.

29. Verfahren nach mindestens einem der Ansprüche 1-3, wobei auf einen Kontaktphase-Aktivator verzichtet wird und die Gerinnung auf Basis der RVVT mit Reagenzien durchgeführt wird, die Faktor X und Faktor V aktivierende Faktoren aus Schlangengift und Phospholipide beinhalten.

30. Verfahren nach Anspruch 29, wobei die Faktor X und Faktor V aktivierenden Faktoren gereinigt oder durch Zugabe von unfraktioniertem Schlangengift dem Reagenz zugesetzt werden.

31. Verfahren nach mindestens einem der Ansprüche 29 und 30, wobei das Faktor X und Faktor V aktivierende Faktoren-haltige Reagenz bereits einen Protein C-Aktivator enthält oder die Zugabe der Faktor X und Faktor V aktivierenden Faktoren getrennt von der Zugabe des Protein C-Aktivators erfolgt.

## Claims

1. A screening test for the detection of disturbances of the protein C/protein S system of clotting in a sample of a biological fluid, which includes the following steps:
a) addition of an activator of protein C to the diluted or undiluted sample, the sample not being mixed with a protein C or protein S deficient plasma;
b) optional addition of a contact phase activator;
c) incubation of the reaction mixture;
d) starting the clotting process by adding calcium ions and/or other agents which induce clotting and
e) determination of the clotting activity.

2. The method as claimed in claim 1, wherein the protein C activator is selected from the group of protein C activators which predominantly or exclusively activate protein C, preferably from the group of snake venom enzymes, particularly preferably the venom from the *Agkistrodon* genus.

3. The method as claimed in at least one of claims 1-2, wherein the method is a modification of a standard method for detecting tendencies to bleeding such as determination of the activated partial thromboplastin time (APTT), of the prothrombin time (PT), or of the Russell's viper venom time (RVVT).

4. The method as claimed in at least one of claims 1-3, wherein a contact phase activator is selected from the group consisting of kaolin, silica, glass and ellagic acid.

5. The method as claimed in at least one of claims 1-4, wherein at least the incubation in step c) takes place at controlled temperatures, preferably at 36-38°C.

6. The method as claimed in at least one of claims 1-5, wherein the concentration of the protein C activator is adjusted so that the clotting time for a normal plasma is prolonged by at least 30%, preferably at least 100%, very preferably at least 200%.

7. The method as claimed in at least one of claims 1-6, wherein the incubation time in step c) is adjusted so that the clotting time for a normal plasma is prolonged by at least 30%, preferably at least 100%, very preferably at least 200%.

8. The method as claimed in at least one of claims 1-7, wherein another incubation takes place between step a) and b).

9. The method as claimed in at least one of claims 1-8, wherein
i) the addition of an activator of protein C,
ii) the addition of a contact phase activator and
iii) the start of the clotting process by adding calcium ions
takes place simultaneously or successively at short time intervals.

10. The method as claimed in at least one of claims 1-9, wherein a chromogenic substrate for determining the clotting activity is used in step e).

11. The method as claimed in at least one of claims 1-10, wherein phospholipids are added to the mixture before step c).

12. The method as claimed in at least one of claims 1-10, wherein phospholipids are added to the mixture after step c).

13. The method as claimed in at least one of claims 11 and 12, wherein phospholipids are selected from the group of phospholipids which bring about the attachment of enzyme/cofactor complexes to the resulting surfaces.

14. The method as claimed in at least one of claims 1-13, wherein the time until a clot detectable by mechanical, mechano-optical or turbidimetric measurement methods is produced is used to determine the clotting activity in step e).

15. The method as claimed in claim 10, wherein the conversion of a chromogenic substrate for thrombin is used for the photometric determination of the clotting activity in step e).

16. A composition for use in a method as claimed in at least one of claims 1-9, where the reagent contains the protein C activator, the contact phase activator and phospholipids in a formulation customary for in vitro diagnosis, wherein the concentration of the protein C activator is chosen so that the clotting time with normal plasmas is prolonged by at least 30%, particularly preferably at least 100%, very particularly preferably at least 200%, compared with the clotting time without the presence of a protein C activator.

17. A test kit for use in a method as claimed in at least one of claims 1-15, which contains at least one composition as claimed in Claim 16.

18. A method wherein with samples which, in a method as claimed in claims 1-3, show differences in the clotting time deviating from normal plasma, the clotting times are determined anew in a method as claimed in claims 1-3, where further components which are suitable for compensating for endogenous deficits in the protein C/protein S system are added in one of steps a) to e).

19. The method as claimed in claim 18, wherein the further components are selected from the group: protein C, protein S, factor V or phospholipids.

20. The method as claimed in claim 19, wherein the proteins employed are of human origin.

21. The method as claimed in claim 19, wherein the proteins employed are of animal origin.

22. The method as claimed in at least one of claims 18-21, wherein the concentration of the further components is selected so that, in a suitable mixture with the sample, they compensate for a disturbance in the protein C/protein S system based on a deficiency or defect in these further components.

23. The method as claimed in claim 22, wherein the concentration of the further components is selected so that after mixing the sample contains that functional amount of substance which is to be found in a normal plasma.

24. The method as claimed in claim 22, wherein the further component is factor V, and the concentration of factor V is chosen so that after mixing the sample contains an at least two-fold excess over the amount of functional factor V which is to be found in a normal plasma.

25. The method as claimed in at least one of claims 18-24, wherein the mixing of the sample with the further component(s) takes place before adding the protein C activator, preferably the sample is incubated with the additional component(s) before adding the protein C activator for a time of 1-10 min.

26. The method as claimed in at least one of claims 1-3, wherein no contact phase activator is used, and the clotting based on the prothrombin time can be induced with reagents which contain tissue factor and phospholipids.

27. The method as claimed in claim 26, wherein the tissue factor can be of natural or recombinant origin.

28. The method as claimed in at least one of claims 26 and 27, wherein the tissue factor-containing reagent already contains a protein C activator, or the addition of the tissue factor takes place separate from the addition of the protein C activator.

29. The method as claimed in at least one of claims 1-3, wherein no contact phase activator is used, and the clotting based on the RVVT is carried out with reagents which contain the factor X and factor V activating factors from snake venom and phospholipids.

30. The method as claimed in claim 29, wherein the factor X and factor V activating factors are purified or are added by adding unfractionated snake venom to the reagent.

31. The method as claimed in at least one of claims 29 and 30, wherein the reagent containing factor X and factor V activating factors already contains a protein C activator, or the addition of the factor X and factor V activating factors takes place separate from the addition of the protein C activator.

## Revendications

1. Test analytique pour la détection de troubles du système protéine C/ protéine S. de la coagulation, dans un échantillon d'un liquide biologique, qui comprend les étapes suivantes:
a) addition d'un activateur de la protéine C à l'échantillon dilué ou non dilué, l'échantillon n'étant pas mélangé avec un plasma déficient en protéine C ou en protéine S ;
b) addition éventuelle d'un activateur de phase de contact:
c) incubation du mélange réactionnel ;
d) déclenchement du processus de coagulation par addition d'ions calcium et/ou d'autres agents déclenchant la coagulation et
e) détermination de l'activité de coagulation.

2. Procédé selon la revendication 1, dans lequel l'activateur de protéine C est choisi dans le groupe des activateurs de protéine C qui activent essentiellement ou exclusivement la protéine C, de préférence dans le groupe des enzymes de venins de serpents, de façon particulièrement préférée, le venin du genre *Agkistrodon.*

3. Procédé selonau moins l'une des revendications 1 et 2, dans lequel le procédé consiste en une modification d'une méthode classique pour la détection de tendances à l'hémorragie, telle que la détermination du temps de céphaline activé (APTT), du temps de céphaline (PT) ou du temps de venin de vipère de Russell (RVVT).

4. Procédé selon au moins l'une des revendications 1 à 3, dans lequel l'activateur de phase de contact est choisi dans le groupe qui comprend le kaolin, la silice, le verre et l'acide ellagique.

5. Procédé selon au moins l'une des revendications 1 à 4, dans lequel au moins l'incubation dans l'étape c) se déroule à des températures réglées, de préférence à 36-38°C.

6. Procédé selon au moins l'une des revendications 1 à 5, dans lequel la concentration de l'activateur de la protéine C est ajustée de manière que le temps de coagulation pour un plasma normal soit augmenté d'au moins 30 %, de préférence d'au moins 100 %, encore mieux d'au moins 200 %.

7. Procédé selon au moins l'une des revendications 1 à 6, dans lequel le temps d'incubation dans l'étape c) est choisi de manière que le temps de coagulation pour un plasma normal soit augmenté d'au moins 30 %, de préférence d'au moins 100 %, encore mieux d'au moins 200 %.

8. Procédé selon au moins l'une des revendications 1 à 7, dans lequel une autre incubation est effectuée entre les étapes a) et b).

9. Procédé selon au moins l'une des revendications 1 à 8, dans lequel
I) l'addition d'un activateur de la protéine C,
II) l'addition d'un activateur de phase de contact et
III) le déclenchement du processus de coagulation par addition d'ions calcium s'effectuent en même temps ou successivement à courts intervalles de temps.

10. Procédé selon au moins l'une des revendications 1 à 9, dans lequel on utilise dans l'étape e) un substrat chromogène pour la détermination de l'activité de coagulation.

11. Procédé selon au môins l'une des revendications 1 à 10, dans lequel on ajoute des phospholipides au mélange avant l'étape c).

12. Procédé selon au moins l'une des revendications 1 à 10, dans lequel on ajoute des phospholipides au mélange après l'étape c).

13. Procédé selon au moins l'une des revendications 11 et 12, dans lequel les phospholipides sont choisis dans le groupe des phospholipides qui provoquent la fixation des complexes enzyme/cofacteur aux surfaces formées.

14. Procédé selon au moins l'une des revendications 1 à 13, dans lequel on utilise pour la détermination de l'activité de coagulation dans l'étape e) le temps jusqu'à la formation d'un caillot détectable par des méthodes mécaniques, mécano-optiques ou turbidimétriques,

15. Procédé selon la revendication 10, dans lequel on utilise pour la détermination photométrique de l'activité de coagulation dans l'étape e) la réaction d'un substrat chromogène pour la thrombine.

16. Composition pour utilisation dans un procédé selon au moins l'une des revendications 1 à 9, dans laquelle le réactif contient l'activateur de la protéine C, l'activateur de phase de contact et des phospholipides dans une préparation usuelle dans le diagnostic in vitro, **caractérisée en ce que** la concentration de l'activateur de la protéine C est choisie de manière que dans des plasmas normaux une augmentation du temps de coagulation par rapport au temps de coagulation sans présence d'un activateur de la protéine C soit d'au moins 30 %, de façon particulièrement préférée d'au moins 100 %, de façon tout particulièrement préférée d'au moins 200 %.

17. Nécessaire d'essai pour utilisation dans un procédé selon au moins l'une des revendications 1 à 15, qui contient au moins une composition selon la revendication 16.

18. Procédé **caractérisé en ce que**, dans des échantillons qui présentent des différences des temps de coagulation qui s'écartent, dans un procédé selon les revendications 1 à 3, du plasma normal, on détermine à nouveau les temps de coagulation dans un procédé selon les revendications 1 à 3, en ajoutant dans l'une des étapes a) à e) d'autres composants qui sont appropriés à compenser des déficits endogènes dans le système protéine C/protéine S.

19. Procédé selon la revendication 18, dans lequel les autres composants sont choisis dans le groupe : protéine C, protéine S, facteur V ou phospholipides.

20. Procédé selon la revendication 19, dans lequel les protéines utilisées sont d'origine humaine.

21. Procédé selon la revendication 19, dans lequel les protéines utilisées sont d'origine animale.

22. Procédé selon au moins l'une des revendications 18 à 21, dans lequel la concentration des autres composants est choisie de manière qu'en mélange approprié avec l'échantillon ils compensent une perturbation du système protéine C/protéine S due à une insuffisance ou à un défaut de ces autres composants.

23. Procédé selon la revendication 22, dans lequel la concentration des autres composants est choisie de manière qu'après mélange soit présente dans l'échantillon la quantité de substances fonctionnelles qui se trouve dans un plasma normal.

24. Procédé selon la revendication 22, dans lequel l'autre composant est le facteur V et la concentration en facteur V est choisie de manière qu'après mélange soit présent dans l'échantillon un excès au moins double de la quantité de facteur V fonctionnel qui peut être trouvée dans un plasma normal.

25. Procédé selon au moins l'une des revendications 18 à 24, dans lequel le mélange de l'échantillon avec l'autre ou les autres composants s'effectue avant l'addition de l'activateur de la protéine C, de préférence l'échantillon est mis à incuber pendant une durée de 1 à 10 minutes avec le ou les composants supplémentaires, avant l'addition de l'activateur de la protéine C.

26. Procédé selon au moins l'une des revendications 1 à 3, dans lequel on se dispense d'un activateur de phase de contact et on peut déclencher la coagulation sur la base du temps de céphaline, avec des réactifs qui comportent un facteur tissulaire et des phospholipides.

27. Procédé selon la revendication 26, dans lequel le facteur tissulaire peut être d'origine naturelle ou recombinante.

28. Procédé selon au moins l'une des revendications 26 et 27, dans lequel le réactif contenant un facteur tissulaire contient déjà un activateur de la protéine C ou l'addition du facteur tissulaire s'effectue séparément de l'addition de l'activateur de la protéine C.

29. Procédé selon au moins l'une des revendications 1 à 3, dans lequel on se dispense d'un activateur de phase de contact et la coagulation s'effectue sur la base de RVVT avec des réactifs qui comportent des facteurs activant le facteur X et le facteur V, provenant de venin de serpent, et des phospholipides.

30. Procédé selon la revendication 29, dans lequel les facteurs activant le facteur X et le facteur V sont purifiés ou ajoutés au réactif par addition de venin de serpent non fractionné.

31. Procédé selon au moins l'une des revendications 29 et 30, dans lequel le réactif contenant des facteurs activant le facteur X et le facteur V contient déjà un activateur de la protéine C ou l'addition des facteurs activant le facteur X et le facteur V s'effectue séparément de l'addition de l'activateur de la protéine C.
